# EUROPEAN PATENT APPLICATION

(11) **EP 4 475 131 A1**
(43) Date of publication of application: **11.12.2024**
(21) Application number: 23315237.0
(22) Date of filing: 05.06.2023
(51) Int. Cl.: G16C 20/30

(54) **PREDICTION OF ODOR PROFILES OF VOLATILE ORGANIC COMPOUNDS BY MACHINE LEARNING**

(71) Applicant: Aryballe, 38000 Grenoble (FR)
(72) Inventor: Singh, Krishna Kant, 38000 Grenoble (FR)
(74) Representative: Schuffenecker, Thierry

(57) **Abstract**

A method for predicting the sensorgram or signature of a volatile organic compound (VOC) using a trained graph neural network is disclosed. A sensorgram is the output of an electronic nose based on Mach-Zehnder interferometers. A signature is a representation of a sensorgram as a radar chart. The method comprises converting the SMILES string of a VOC to a graph comprising nodes and edges. The graph is used as input to a trained graph neural network that outputs a predicted signature of the VOC.

## Description

### Technical field

The document provides examples of signal processing, in particular machine learning in the technical domain of sensors for odors, e.g., electronic noses.

### Background

An "odor" (American English) or "odour" (British English) is caused by Volatile Organic Compounds (VOCs) and/or molecules emitted by objects in the environment, which can be found in low concentrations in the air that humans and animals can perceive by their sense of smell. Other terms with different connotations can be used e.g., "smell", "scent", "aroma", "fragrance", "perfumes", etc.

In nature, volatile organic compounds (VOCs) are organic chemicals that easily evaporate at room temperature and are released into the air. The sheer number and complexity of VOCs present in nature make it a challenging task to generate their odor profiles using an electronic nose. An electronic nose is a device designed to mimic the olfactory system of humans or other animals, enabling it to detect and analyze odors. It consists of a sensor array that responds to different chemical compounds and a pattern recognition system that interprets the sensor responses to identify and classify the odors.

When it comes to generating the odor profiles of individual VOCs, several challenges arise. First, there are millions of different VOCs found in nature, each with its unique chemical structure and odor characteristics. Capturing and representing the vast diversity of VOCs within a sensor array is a significant manual and technical hurdle.

The challenges become even more pronounced when attempting to generate odor profiles of mixtures of VOCs.

An electronic nose based on biomolecules is a type of sensor system that uses biological molecules such as peptide, enzymes, antibodies, or nucleic acids to detect and identify specific volatile organic compounds (VOCs). This approach is inspired by the natural olfactory system, where the binding of odorant molecules to receptor proteins in the nose triggers a neural response that is interpreted as an odor.

In bio-molecule-based electronic noses, the biological molecules are immobilized onto a sensor surface and are designed to selectively bind to specific VOCs. When the VOCs come into contact with the sensor surface, the binding of the molecules to the VOCs causes a change in the electrical or optical properties of the sensor, which can be measured and analyzed to identify the odor.

In a peptide-based electronic nose, the peptides are immobilized onto a sensor surface. The utilization of peptides as sensors in electronic noses offers several advantages. Peptides can exhibit remarkable selectivity, as their sequence and three-dimensional structure can be tailored to recognize specific VOCs with high precision. These peptides are carefully designed or selected to have high affinity and selectivity for certain VOCs.

These computational techniques such as Molecular and quantum simulations can be used to gain valuable insights into the interactions between VOCs and peptides, aiding in the understanding and prediction of odor profiles. However, these approaches have significant limitations while studying volatile organic compounds (VOCs) in the gas phase. Therefore, estimating the odor profile of volatile organic compounds (VOCs) using quantum mechanical (QM) and molecular mechanics (MM) techniques is very challenging. Moreover, generating odor profiles of mixtures of VOCs is even more challenging. This patent presents data-driven approaches for estimating the odor profile of volatile organic compounds (VOCs).

The article entitled "SMILE to smell" was published on January 15, 2021 in J. Chem. Inf. Model. 2021, 61, 2, 676-688 converts SMILES (simplified molecular-input line-entry system values) into images and uses CNN networks. According to the article, finding the relationship between the structure of an odorant molecule and its associated smell has always been an extremely challenging task. The major limitation in establishing the structure-odor relation is the vague and ambiguous nature of the descriptor-labeling, especially when the sources of odorant molecules are different. With the advent of deep networks, data-driven approaches have been substantiated to achieve more accurate linkages between the chemical structure and its smell. In this study, the deep neural network (DNN) with physicochemical properties and molecular fingerprints (PPMF) and the convolution neural network (CNN) with chemical-structure images (IMG) are developed to predict the smells of chemicals using their SMILES notations. A data set of 5185 chemical compounds with 104 smell percepts was used to develop the multilabel prediction models. The accuracies of smell prediction from DNN + PPMF and CNN + IMG (Xception based) were found to be 97.3 and 98.3%, respectively, when applied on an independent test set of chemicals. The deep learning architecture combining both DNN + PPMF and CNN + IMG prediction models is proposed, which classifies smells and may help understand the generic mechanism underlying the relationship between chemical structure and smell perception.

This approach is interesting but presents limitations. Inter alia, it leads to relatively bad results for odor profiles predictions.

The blog article entitled "Digitizing Smell: Using Molecular Maps to Understand Odor" published on September 6th 2022 by Richard C. Gerkin and al., at Google Research teaches how to use graph neural network (GNN) models but nothing more.

The patent document US20220139504 entitled "Systems and Methods for Predicting the Olfactory Properties of Molecules Using Machine Learning" describes systems and methods for predicting olfactory properties of a molecule. One example method includes obtaining a machine-learned graph neural network trained to predict olfactory properties of molecules based at least in part on chemical structure data associated with the molecules. The method includes obtaining a graph that graphically describes a chemical structure of a selected molecule. The method includes providing the graph as input to the machine-learned graph neural network. The method includes receiving prediction data descriptive of one or more predicted olfactory properties of the selected molecule as an output of the machine-learned graph neural network. The method includes providing the prediction data descriptive of the one or more predicted olfactory properties of the selected molecule as an output. This approach presents limitations.

There is a need for methods and systems for predicting odor profiles of Volatile Organic Compounds using machine learning.

### Summary

There is described a computer-implemented method comprising the steps of: receiving data from one or more electronic noses; representing a plurality of known pure Volatile Organic Compounds (VOCs) as a plurality of simplified molecular-input line-entry system values (SMILEs); converting said plurality of simplified molecular-input line-entry system values into a graph comprising nodes and edges; assigning features to nodes and edges; training a multi-layer Graph Neural Network on a dataset of graphs of simplified molecular-input line-entry system values. Developments describe the use of a Graph Neural Network; intensity, association and/or dissociation constants, weight by vapor pressure, the use of graphs indicating the relationship between Volatile Organic Compounds and signatures. Hardware aspects comprise the use of one or more of: a Surface Plasmon Resonance (SPR), a Mach-Zehnder interferometer (MZI), MEMS (Micro-electro-mechanical systems), Capacitive Micromachined Ultrasonic Transducer (CMUT), Aligned Carbon Nanotubes (CNTs), peptides, polymers, piezo resistance mechanisms and/or living cells.

A peptide-based electronic nose utilizes sensorgrams to capture the interaction between volatile organic compounds (VOCs) and peptides immobilized on a sensor surface. A sensorgram is a graphical representation that shows changes in the sensor's response, such as electrical or optical signals, over time when exposed to VOCs.

When a VOC interacts with the peptide-based sensor, it induces specific binding events, resulting in unique sensorgram patterns or signatures. These signatures represent the characteristic response of the sensor to different VOCs. By analyzing the sensorgram patterns, it is possible to extract distinctive signatures (or odor profiles) that can be associated with specific VOCs. To represent VOCs in a format suitable for analysis, the Simplified Molecular input Line Entry System (SMILES) notation can be used. SMILES is a compact and linear textual representation of a molecule's structure. It encodes the connectivity and arrangement of atoms in a molecule, allowing for easy comparison and analysis.

Advantageously, while there are no SMILES for wine smelling, some embodiments allow to handle data of N pure VOCs composing the wine, said pure VOCs interfering with each other and leading to signatures of mixtures of VOCs. Then, odor profiles can be derived from said signatures. Such simulations can greatly help wine makers and tasters.

Advantageously, some embodiments can be applied in the food industry (for example when opening a package, malodor or spoilage detection).

Advantageously, signatures or odor profiles of mixtures of VOCs are not available in databases. Thanks to some embodiments, consisting in data augmentation (for example at different concentration levels), it becomes possible to get such data and to predict signatures of mixtures of VOCs.

Advantageously, some embodiments allow embedding computations in processors with very low computing capacity.

Various deep learning systems have been tried. As these systems are mostly black boxes, very diverse results can be obtained. For example, CNN deep learning performed on images does not provide satisfactory results. Handling NLP on text strings representing SMILES also does not provide good results.

Surprisingly, handling graphs derived from SMILES data provides good predictions.

According to some embodiments, SMILES are advantageously transformed into learnable embeddings using deep learning. These embeddings are used to train a model that predicts odor profile (aka signatures).

If data is provided in table forms, then various systems can be tried, for example but not limited to Support-vector machines, Linear regression, Logistic regression, Naive Bayes, Linear discriminant analysis, Decision trees, K-nearest neighbor algorithm, Neural networks (Multilayer perceptron) and similarity learning for example.

SMILES exist only for pure VOCs. Signatures do not vary. Odor profiles associated with signatures can evolve (depending on humidity or other environmental conditions e.g., temperature).

With probabilistic models : images-based models do not work well. Random forest techniques do not work well either. Graph based models work surprisingly well.

Advantageously, artificial intelligence techniques can create functions that map SMILE into signatures by using data captured by an electronic nose.

### Brief description of drawings

Embodiments of the present invention will now be described by way of example with reference to the accompanying drawings in which like references denote similar elements, and in which:
FIG. 1 illustrates an embodiment of an electronic nose.
FIG. 2 illustrates the representation of molecules as graphs and the use of SMILES.
FIG. 3 and 4 illustrates the prediction of a model according to an embodiment and low error rate thereof.
FIG. 5 and 6 illustrates the influence of vapor pressure.
FIG. 7 illustrates the odor profiles of different molecules.
FIG. 8 illustrates predicted signatures.
FIG. 9 illustrates an overview of relationships between VOCs, signatures, odor space, chemical space and smell.

### Detailed description

### Definitions

### Sensorgrams

The electronic nose works by exposing the chemical sensors to a sample of air or gas, which contains the odor or VOCs of interest. The sensors then produce a signal that is analyzed to identify the specific odors or VOCs present in the sample. The output of the electronic nose is typically a pattern or signature of the different odors or VOCs present in the sample. E-nose output typically consists of sensorgrams, which are time-varying patterns representing the response of individual sensors in the sensor array. These sensorgrams provide information about the changes in electrical signals produced by the sensors when exposed to different volatile compounds.

### Signatures

Signatures are dependent on environmental properties such as humidity and temperature.

To extract signatures from the sensorgrams, additional data processing techniques are employed. The signatures represent characteristic features or patterns derived from the sensorgram data that are specific to different chemical compounds or chemical families. The sensorgram, which is a temporal sequence of sensor responses, is transformed or processed to derive a more condensed and informative signature. Most common approach to generate a signature from a sensorgram is by taking the average of the sensor responses over time. This method involves calculating the mean value of each sensor's response across the entire or defined duration of the sensorgram.

The average-based signature provides a compact representation of the overall sensor response pattern over time. It captures the central tendency of the sensorgram data and smooths out any short-term fluctuations or noise.

Once the signatures are extracted, they provide a condensed representation of the sensorgram data, highlighting the essential characteristics associated with different chemical compounds or families. These signatures can be used for pattern recognition, classification, or identification tasks. Machine learning algorithms can be trained on the extracted signatures, allowing the electronic nose to recognize and differentiate between different chemical compounds or families based on their sensorgram patterns.

By combining the sensorgram output of an electronic nose with the extraction of signatures, it becomes possible to capture the distinctive features of volatile compounds and enhance the accuracy and reliability of chemical identification and classification.

There is described a computer-implemented method comprising the steps of: receiving sensorgram data from one or more electronic noses; representing a variety of known pure Volatile Organic Compounds, or VOCs, as simplified molecular-input line-entry system values, or SMILES; converting the collection of SMILES values into a graph representation comprising nodes and edges; assigning relevant features to the nodes and edges of the graph; building a comprehensive database that incorporates SMILES, graphs, sensorgrams, and signatures for the VOCs; training a multi-layer Graph Neural Network using a dataset where the input consists of graphs with simplified molecular-input line-entry system values, and the output corresponds to signatures.

In a development, the method further comprises using one or more trained Graph Neural Networks to predict the signature of a newly received Volatile Organic Compound.

In a development, obtaining a predicting model GNN comprises: building a database of Volatile Organic Compounds' signatures using an electronic nose in gas phase; training a machine learning system, or one or more Graph Neural Networks to predict a sensorgram from the structure of a Volatile Organic Compound;
In a development, obtaining a predicting model with a Graph Neural Networks comprises to predict both signature and sensorgram.

In a development, SMILES are trained to predict properties of VOCs comprising at least one of intensity of smell, association constants or dissociation constants with sensors.

In a development, predicting the signature of a mixture of Volatile Organic Compounds is performed by : predicting signature of a received individual Volatile Organic Compound; determining the signature of a mixture of Volatile Organic Compounds by summing individual Volatile Organic Compound signatures weighted by their vapor pressure;

In a development, a predicted signature is a weighted average of concentration of Volatile Organic Compounds and of vapor pressure.

In a development, one of the Volatile Organic Compounds is water or humidity.

In a development, the method further comprises the step of predicting a sensorgram of a mixture.

In a development, the method further comprises determining data indicative of how a structural change of a VOC affects the signature.

In a development, a graph that graphically describes the structure of a Volatile Organic Compound comprises a low-dimensional embedding of a graph structure indicating the relationship between Volatile Organic Compound and its signature.

There is a system configured to perform one or more steps of the method.

In a development, one or more physical and/or biological sensors are configured to measure data ; and/or one or more computer processors configured to process said measured data.

In a development, a sensor comprises one or more Mach-Zehnder interferometers.

In a development, a sensor comprises one or more capacitive micromachined ultrasonic transducers.

In a development, a sensor comprises one or more of an enzyme, antibody, sugar, fatty acid or nucleic acid.

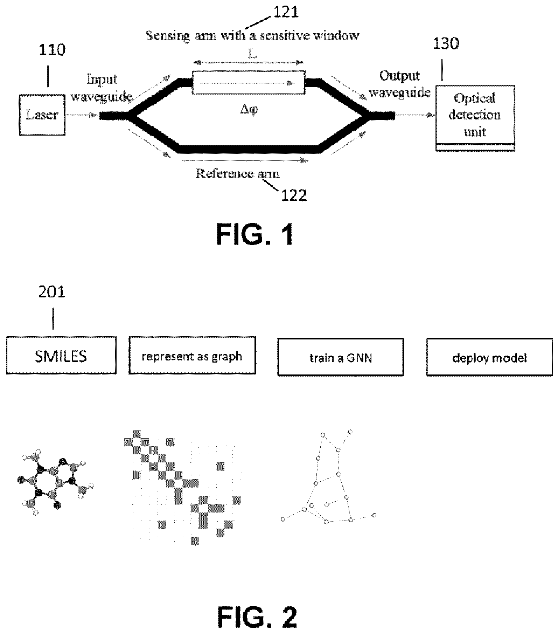

FIG. 1 illustrates an embodiment of an electronic nose.

An electronic nose is a sophisticated sensing device that emulates the human olfactory system to detect and analyze odors or volatile compounds in the surrounding environment. It consists of an array of chemical sensors that respond to specific odor molecules or volatile compounds. When exposed to an odor, the sensors generate a unique pattern of responses, known as a sensorgram.

A peptide-based electronic nose is a sensor technology that utilizes peptides as sensing elements to detect and discriminate volatile organic compounds (VOCs) based on their odor characteristics. When VOCs interact with the peptides immobilized on the sensor surface, they trigger specific binding events. The resulting sensor response, which can be measured as electrical or optical signals, is captured as a sensorgram. A sensorgram is a graphical representation that illustrates the changes in the sensor's response over different phases of interaction, including the adsorption phase when VOCs initially interact with the peptides, the stabilization phase where the binding affinity reaches equilibrium, and the desorption phase when the VOCs are released from the sensor surface.

In the context of an electronic nose, the term "signature" (or odor profile) refers to a consolidated representation of the sensorgram data that captures the distinctive characteristics of an odor or volatile compound. The sensorgram, which is a temporal sequence of sensor responses, is transformed or processed to derive a more condensed and informative signature. Most common approach to generate a signature from a sensorgram is by taking the average of the sensor responses over time. This method involves calculating the mean value of each sensor's response across the entire or defined duration of the sensorgram.

The average-based signature provides a compact representation of the overall sensor response pattern over time. It captures the central tendency of the sensorgram data and smooths out any short-term fluctuations or noise. This approach is particularly useful when the sensor responses exhibit consistent trends or when the focus is on the general characteristics of the odor rather than specific temporal dynamics.

The structure of an electronic nose according to some embodiments can be diverse.

In an embodiment, the "electronic nose" or "analyzing system" comprises a measurement chamber 121 intended to receive the target compounds contained in either gas, liquid or fluid sample, wherein a plurality of separate sensitive sites, each comprising receivers able to interact with the target compounds, is located in said chamber.

In an electronic nose, VOCs (or fluid sample) are generally injected (passively and/or actively) into a chamber 121 with or without inert gas (e.g., argon). Distinct sensor units (of distinct chemical affinity) forming a "multivariate sensor" in the chamber then react to compounds or VOCs or molecules of the fluid sample and from an analog output, a digital output is determined, and further interpreted. The digital output called "sensorgram" typically contains four phases: 1. Baseline 2. adsorption 3. equilibrium and 4. desorption.

Databases can be constructed using various representations, including sensorgrams, signatures, and low-dimensional embeddings, to capture and store information about volatile organic compounds (VOCs). Furthermore, other Sensorgrams, which are graphical representations of the sensor's response over time, can be recorded and stored as part of a database. These sensorgrams serve as a valuable source of information about the interaction between VOCs and the sensor. In addition to sensorgrams and signature data, computed association and dissociation constants can be included in the database. These constants represent the strength of the binding between VOCs and the sensor elements. Computation of these constants provides quantitative insights into the VOC-sensor interactions and enables the estimation of binding affinity.

In an embodiment, the sensor comprises bio-molecules (such as peptide/protein/Nucleic acid etc. that interact with plurity of VOC/VOCs) and an optical integrated circuit comprising: a lower layer called substrate; a first coupling means adapted to couple a radiation incident light (laser) 100 to the integrated optical circuit; a directional splitter connected to the first coupling means and configured to separate the light radiation coupled by the first means from the second means coupling to at least one pair of waveguides comprising: a first waveguide known as a sensitive arm 121 in which is propagated a first portion of the light radiation, said sensitive arm 121 being exposed to a first ambient medium and at least one compound to be detected that induces a modification of the local refractive index perceived by the evanescent part of the electromagnetic field of the first portion of the light radiation, and a second waveguide called a reference arm 122 in which is propagated a second portion of the light radiation, an encapsulation layer encapsulating the reference arm, said layer being impermeable to the compound(s) to be detected, so as to be able to detect that the reference arm is exposed only to a second medium the same nature as the first ambient environment, and devoid of said compound to be detected ; a directional combiner combining the first portion of the radiation from said reference arm, called the first transmitted portion, and the second portion of the light radiation from said sensitive arm, called the "sensitive arm" second transmitted portion, to form a transmitted radiation ; second coupling means adapted to couple said radiation; and transmitted to a medium external to the integrated optical circuit; an upper layer called superstrate covering at least the first and the second layers of the optical integrated circuit; second coupling means, the directional separator and the combiner directional and does not cover the sensitive and reference arm, said encapsulation layer being deposited on top of the superstrate.

In an embodiment, the sensor comprises: biomolecules grafted on a surface and a laser source 100 configured to emit incident light radiation; an integrated optical circuit; an optical detection system 130 adapted to detect a light radiation; - an integrated optical circuit from the second coupling means and generate a signal representative of the evolution over time of the detected light intensity; a unit for processing the said signal, adapted to determine, from the intensity detected, the evolution over time of the phase shift between the first portion and the second portion transmitted and the second portion transmitted.

Sensors in modern technology are becoming more and more complex. In some embodiments, an electronic nose can comprise one or more of SPR, MZI, MEMS, CMUT, and/or Carbon Nanotubes.

### Surface Plasmon Resonance (SPR)

Surface plasmon resonance (SPR) occurs where electrons in a thin metal sheet become excited by light that is directed to the sheet with a particular angle of incidence, and then travel parallel to the sheet. Assuming a constant light source wavelength and that the metal sheet is thin, the angle of incidence that triggers SPR is related to the refractive index of the material and even a small change in the refractive index will cause SPR to not be observed. This makes SPR a possible technique for detecting particular substances (analytes) and SPR biosensors have been developed to detect various important biomarkers

### Mach-Zehnder interferometer (MZI)

Response signals of a MZI transducer (Mach-Zehnder Interferometer) are signals modulated (by an optical stage) in frequency proportional to the difference in step between the wave in the reference guide and the wave in the measuring guide. This difference allows the reconstruction of an odor sensorgram. In variants, the multivariate sensor comprises a Fizeau interferometer or a Fabry-Perot interferometer or a Jamin interferometer or a Ramsey-Bordé interferometer.

### MEMS

MEMS (Micro-electro-mechanical systems) incorporate both electronic and moving parts. MEMS are made up of components between 1 and 100 micrometers in size, and MEMS devices generally range in size from 20 micrometers to a millimeter, although components arranged in arrays (e.g., digital micromirror devices) can be more than 1000 mm2. They usually consist of a central unit that processes data (an integrated circuit chip such as microprocessor) and several components that interact with the surroundings (such as microsensors)

### Capacitive Micromachined Ultrasonic Transducer, acronym CMUT

In an embodiment, the multivariate sensor comprises a Capacitive Micromachined Ultrasonic Transducer, acronym CMUT. CMUTs are the transducers where the energy transduction is due to change in capacitance. CMUTs are constructed on silicon using micromachining techniques. A cavity is formed in a silicon substrate, and a thin layer suspended on the top of the cavity serves as a membrane on which a metallized layer acts as an electrode, together with the silicon substrate which serves as a bottom electrode. In an embodiment, the multivariate sensor can comprise a large number of CMUTs. In particular, CMUT-on-CMOS technology along with a flip-chip process can allow integration of CMUTs with front-end electronics.

### Carbon Nanotubes

Aligned Carbon Nanotubes (CNTs) can be grown at low temperature (250 °C) using Plasma Enhanced Chemical Vapor Deposition (PECVD). CNTs can then be used to identify VOCs in an improved sensitivity condition to gas at room temperature. Sensitivity and stability of carbon nanotube sensors can be improved via growing Titanium Dioxide Nanowires (TiO2-NW) on the surface of CNTs through a hydrothermal method. A threefold increase in sensitivity and a 30-second decrease in response time can be observed as a result of the CNT-TiO2 sensor compared to a pristine CNT sensor.

### Other sensor embodiments

In an advantageous embodiment, peptides are used. Peptides are short chains of between two and fifty amino acids, linked by peptide bonds. Chains of fewer than ten or fifteen amino acids are called oligopeptides, and include dipeptides, tripeptides, and tetrapeptides. A "polypeptide" is a longer, continuous, unbranched peptide chain of up to approximately fifty amino acids. Hence, peptides fall under the broad chemical classes of "biological polymers and oligomers", alongside nucleic acids, oligosaccharides, polysaccharides, and others.

In some embodiments, polymers (on silicon or derivative materials like SiO2 or other traditional materials in micromachining MEMS or silicon photonics) can be used. Silicon is a chemical element with the symbol Si and atomic number 14. It is a semiconductor.

In some embodiments, piezo resistance mechanisms can be used. In some embodiments, living cells can be used. In some embodiments, combinations of the above means can be used, e.g., a combination of peptides and polymers. Depending on use cases (i.e., general purpose or specific detection), the number and/or types of sensor units are diverse.

FIG. 2 illustrates the representation of molecules as graphs and the use of SMILES.

Molecules are the building blocks of matter, and are built of atoms and electrons in 3D space. All particles are interacting, but when a pair of atoms are stuck in a stable distance from each other, we say they share a covalent bond. Different pairs of atoms and bonds have different distances (e.g., single-bonds, double-bonds). VOCs are small organic volatile molecules that can be represented as SMILES (in 1-D) and graphs in 3-D. In general, VOCs have high vapor pressure.

It's a very convenient abstraction to describe this 3D object as a graph, where nodes are atoms and edges are covalent bonds.

In an embodiment, pure VOCs are represented as SMILES 201, acronym of "simplified molecular-input line-entry system".

The simplified molecular-input line-entry system (SMILES) is a specification in the form of a line notation for describing the structure of chemical species using short ASCIIstrings. SMILES strings can be imported by most molecule editors for conversion back into two-dimensional drawings or three-dimensional models of the molecules.

"SMILE" is a binary data format that defines a binary equivalent of standard JSON data format. In other words, SMILE is a computer data interchange format based on JSON. It can also be considered a binary serialization of the generic JSON data model, which means tools that operate on JSON may be used with SMILE as well, as long as a proper encoder/decoder exists for the tool. Compared to JSON, SMILE is both more compact and more efficient to process (both to read and write). Part of this is due to more efficient binary encoding (similar to BSON, CBOR and UBJSON), but an additional feature is optional use of back references for property names and values. Back referencing allows replacing of property names and/or short (64 bytes or less) String values with 1- or 2-byte reference IDs. The syntax for a SMILES structure is a single line of text with no white space. A structure name or identifier can be added to the SMILES string by including it on the same line following the SMILES string and separated by a space, e.g.: C1=CC=CC=C1 benzene

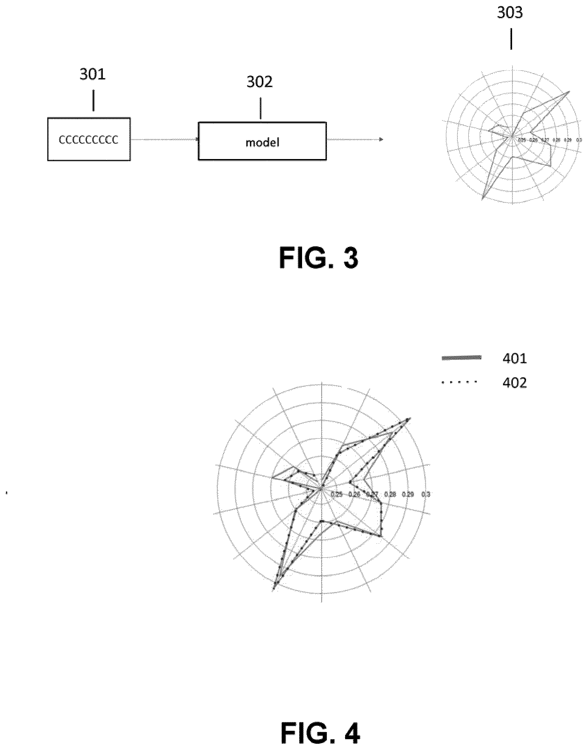

FIG. 3 and 4 illustrates the prediction of a model according to an embodiment and low error rate thereof.

A Graph Neural Network (GNN) is a class of artificial neural networks for processing data that can be represented as graphs. In the more general subject of "Geometric Deep Learning", certain existing neural network architectures can be interpreted as GNNs operating on suitably defined graphs. Convolutional neural networks, in the context of computer vision, can be seen as a GNN applied to graphs structured as grids of pixels. The key design element of GNNs is the use of pairwise message passing, such that graph nodes iteratively update their representations by exchanging information with their neighbors. Since their inception, several different GNN architectures have been proposed, which implement different flavors of message passing. A GNN is an optimizable transformation on all attributes of the graph (nodes, edges, global-context) that preserves graph symmetries (permutation invariances). In an embodiment, a GNN can use the "message passing neural network" framework proposed by Gilmer et al. using the Graph Nets architecture schematics introduced by Battaglia et al. GNNs can adopt a "graph-in, graph-out" architecture, meaning that these model types accept a graph as input, with information loaded into its nodes, edges and global-context, and progressively transform these embeddings, without changing the connectivity of the input graph.

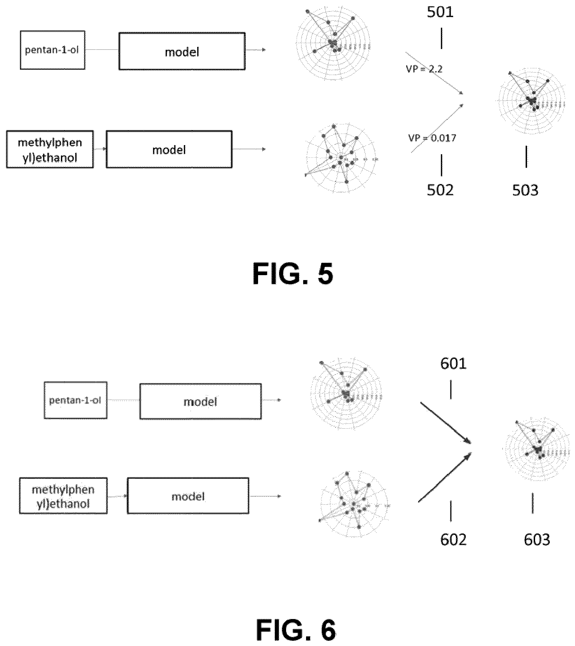

FIG. 5 and 6 illustrates the influence of vapor pressure.

If vapor pressures are known (for example 501 and 502), the predicted signature 503 can be determined. If vapor pressures are not known, it can be assumed that VP=1 (601 and 602), determining predicted signature 603.

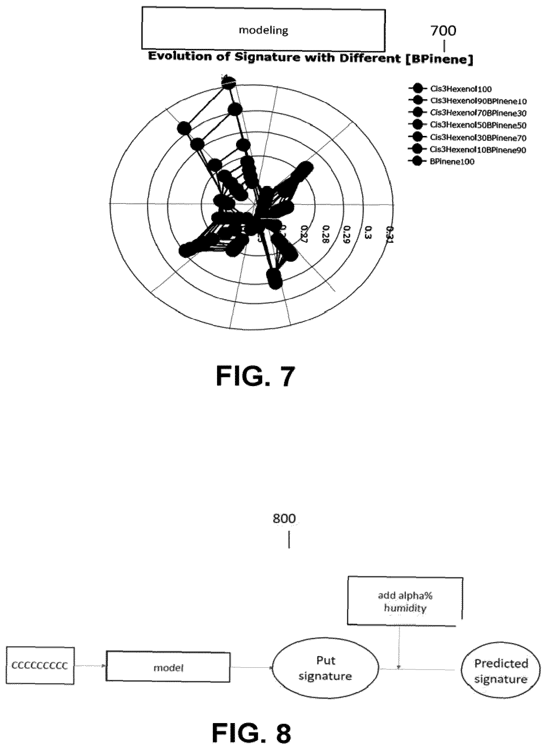

FIG. 7 illustrates the odor profiles of different molecules.

In the example, there is shown the evolution of a signature 700 in different mixtures.

FIG. 8 illustrates predicted signatures.

The figure shows an example of a pipeline for data processing, from SMILES to signature..

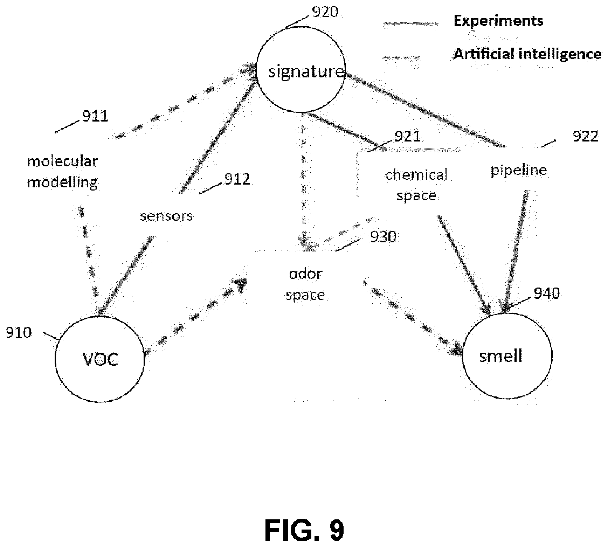

FIG. 9 illustrates an overview of relationships between VOCs, signatures, odor space, chemical space and smell.

VOC 910 designates Volatile Organic Compounds. VOCs are represented as SMILES in theoretical and computational chemistry. These smiles are then used to identify the structure-to-activity relationship also known as QSAR (Quantitative Structural Activity Relationship). In some embodiments, the activity can be the smell associated with a VOC.

Latent low-dimensional space can show a fuzzy relationship between SMILES and smell is known as Odor-space 930.

Most theoretical work is designed for determining relationships between VOC (910), Odor Space 930 and smell 940

In reality, most often, emitted VOCs 910 are not known (so as the composition of VOCs in the gas phase). For example, VOCs are released by wine or perfume. In general, we do not know these VOCs.

Instruments such as electronic nose convert emitting VOC (or mixture of VOCs) into a numerical signal. We refer to this numerical signal as a Signature 920.

Sensors 912 are biological-based multivariate sensors that convert the binding of VOCs with peptide to numerical data using optical methods. For a VOC, the ranking of sensors is directly associated with binding strength.

Molecular modeling 911 approaches such as docking and molecular dynamics can be used to estimate the binding energy of a VOC with a given peptide sensor. Drawbacks comprise the fact that all the biological molecular modeling simulation is validated in the liquid phase and the electronic nose works in the gas phase.

Signatures to smell (922), according to some embodiments, can determine a mapping to convert signatures to smell.

The path 921 (Signature-Chemical Space-Odor) can require a plurality of processes: a) mapping signature to Chemical functional group (example: alcohol, aldehyde, ketone etc.), and mapping Chemical space to odor. It is also possible to map Chemical-space to Odor-space.

There is described a computer-implemented method comprising the steps of: representing a plurality of pure Volatile Organic Compounds as a plurality of simplified molecular-input line-entry system values ; converting said plurality of simplified molecular-input line-entry system values into one or more graphs comprising nodes and edges; determining a signature (binding affinity, for example represented as a radar chart) of a pure VOC by using a probabilistic model that predicts odor profile of a pure VOC. In a development, the probabilistic model is trained by using an attention-based model. In a development, the attention-based model comprises one or more Graph Neural Networks (Graph convolutional network, Graph attention network, Gated graph sequence neural network). In a development, the method further comprises predicting signatures of individual VOC (ex: emitting VOCs, alcohol, smell lavender) based on vapor pressure.; one of the VOC can be humidity/water. In a development, the method further comprises predicting signatures of mixtures weighted based on individual VOC (a+b+c) based on vapor pressure. The weight can be based on vapor pressure (property of VOC cannot be changed) and/or dilution / concentration of vapor pressure. In a development, humidity is an external environmental factor, controllable (in a lab condition) but is otherwise non controllable (like temperature). The signature of water designates humidity. Conditions can be geographically determined. In a development, affine transformations with normalized vapor pressures of VOCs can be used. In a development, the method further comprises determining odor profiles associated with mixtures of pure VOCs based on said signatures, by taking into account different dilution or concentration levels. In a perspective, some embodiments constitute a "data augmentation technique" going from one molecule to a mixture of different molecules in different concentrations. So, with one VOC, it is possible to generate more data at different a) concentration levels and of b) humidity levels

The disclosed methods can take the form of an entirely hardware embodiment (e.g., FPGA), an entirely software embodiment or an embodiment containing both hardware and software elements. Software embodiments include but are not limited to firmware, resident software, microcode, etc. The invention also can take the form of a computer program product accessible from a computer-usable or computer-readable medium providing program code for use by or in connection with a computer or any instruction execution system. A computer-usable or computer-readable can be any apparatus that can contain, store, communicate, propagate, or transport the program for use by or in connection with the instruction execution system, apparatus, or device. The medium can be an electronic, magnetic, optical, electromagnetic, and/or semiconductor system (or apparatus or device) or a propagation medium or the like.

There is disclosed a computer program comprising instructions for carrying out one or more of the steps of the method when said computer program is executed on a computer device.

## Claims

1. A computer-implemented method comprising the steps of:
- receiving sensorgram data from one or more electronic noses;
- representing a variety of known pure Volatile Organic Compounds, or VOCs, as simplified molecular-input line-entry system values, or SMILES;
- converting the collection of SMILES values into a graph representation comprising nodes and edges;
- assigning one or more features to the nodes and edges of the graph;
- building a database that incorporates SMILES, graphs, sensorgrams, and signatures for the VOCs;
- training a machine learning module using a dataset where the input consists of graphs with simplified molecular-input line-entry system values, and the output corresponds to signatures.

2. The computer-implemented method of Claim 1 wherein the machine learning module comprises one or more trained Graph Neural Networks to predict the signature of a newly received Volatile Organic Compound.

3. The computer-implemented method of claim 2, wherein obtaining a predicting model GNN comprises:
- building a database of Volatile Organic Compounds' signatures using an electronic nose in gas phase;
- training a machine learning system, or one or more Graph Neural Networks to predict a sensorgram from the structure of a Volatile Organic Compound.

4. The computer-implemented method of claims 2 and 3 wherein the step of obtaining a predicting model with a Graph Neural Networks comprises the step of to predict both signature and sensorgram.

5. The computer-implemented method of claim 4, wherein SMILES are trained to predict properties of VOCs comprising at least one of intensity of smell, association constants or dissociation constants with sensors.

6. The computer-implemented method of any preceding claim for predicting the signature of a mixture of Volatile Organic Compounds by :
- predicting signature of a received individual Volatile Organic Compound; and
- determining the signature of a mixture of Volatile Organic Compounds by summing individual Volatile Organic Compound signatures weighted by their vapor pressure.

7. The computer-implemented method of claim 4, wherein a predicted signature is a weighted average of concentration of Volatile Organic Compounds and of vapor pressure.

8. The computer-implemented method of claim 7, wherein one of the Volatile Organic Compounds is water or humidity.

9. The computer-implemented method of any one of the claims 6 to 8, further comprising the step of predicting a sensorgram of a mixture.

10. The computer-implemented method of any preceding claim, further comprising determining data indicative of how a structural change of a VOC affects the signature.

11. The computer-implemented method of claim 1, wherein a graph that graphically describes the structure of a Volatile Organic Compound comprises a low-dimensional embedding of a graph structure indicating the relationship between Volatile Organic Compound and its signature.

12. The computer-implemented method of Claim 1 wherein the machine learning module comprises one or more of Supervised Learning, such as one or more of linear regression, logistic regression, decision trees, random forests, support vector machines, and neural networks; Unsupervised Learning, such as k-means, hierarchical clustering, dimensionality reduction, principal component analysis or t-SNE ; Reinforcement Learning, such as Q-learning, Deep Q-Networks, and Proximal Policy Optimization ; Deep Learning, such as Convolutional Neural Networks, Recurrent Neural Networks (RNN) ; and Transfer Learning.

13. A system configured to perform the steps of the method according to any claims 1 to 12.

14. The system of Claim 13, wherein:
- one or more physical and/or biological sensors are configured to measure data ;
- one or more computer processors are configured to process said measured data.

15. The system of Claim 14, wherein a sensor comprises one or more Mach-Zehnder interferometers.

16. The system of Claim 14, wherein a sensor comprises one or more capacitive micromachined ultrasonic transducers.

17. The system of any Claims 13 to 16, wherein the sensor comprises one or more of an enzyme, antibody, sugar, fatty acid or nucleic acid.
